# EUROPEAN PATENT SPECIFICATION

(11) **EP 3 714 936 B1**
(45) Date of publication and mention of the grant of the patent: **30.03.2022**
(21) Application number: 18880318.3
(22) Date of filing: 23.11.2018
(51) Int. Cl.: A61N 1/05, A61N 1/08, A61F 2/24, A61B 17/34, A61B 17/04, A61N 1/39, A61N 1/372, A61N 1/375

(54) **CERCLAGE ATRIAL DEFIBRILLATOR**
CERCLAGE-VORHOFDEFIBRILLATOR
DÉFIBRILLATEUR AURICULAIRE DE CERCLAGE

(30) Priority: 24.11.2017 KR 20170158427
(43) Date of publication of application: 30.09.2020
(73) Proprietor: Tau-PNU Medical Co., Ltd., Busan 46241 (KR)
(72) Inventor: KIM, June Hong, Busan 48516 (KR); CHON, Minku, Yangsan-si Gyeongsangnam-do 50658 (KR); JUNG, Sujin, Yangsan-si Gyeongsangnam-do 50601 (KR)
(74) Representative: dompatent von Kreisler Selting Werner - Partnerschaft von Patent- und Rechtsanwälten mbB
(86) International application number: PCT/KR2018/014556
(87) International publication number: WO 2019/103540

(56) References cited:
- WO-A1-2019/046205
- KR-A- 20150 144 568
- KR-A- 20150 144 568
- KR-B1- 101 563 172
- US-A- 5 978 705
- US-A1- 2010 087 888
- US-A1- 2011 009 957
- US-A1- 2014 114 371
- US-A1- 2016 022 998

## Description

### Technical Field

The present invention is set out in appended claim 1, and generally relates to a cerclage atrial defibrillator and, more particularly, is for treating mitral valve regurgitation and atrial fibrillation by reducing the size of the left atrium through a cerclage wire and treating residual atrial fibrillation by including a defibrillator lead.

### Background Art

The heart consists of two atria and two ventricles. The heart creates an electrical stimulus from electrical cells capable of beating by themselves, and the stimulus is transmitted to the cardiac muscle cells to enable the atria and the ventricles to repeat regular contractions and relaxations, whereby the necessary blood is supplied from the atria to the ventricles, and then from the ventricles to each organ and tissue.

Among tachyarrhythmia, atrial fibrillation is a type of tachycardia that occurs in the atrium, and refers to an arrhythmic disease in which various parts of the atrium vibrate finely, very rapidly, and irregularly, thereby generating irregular pulses. In general, a regular electrical signal is transmitted into the atrium and the atrium contracts and expands regularly. Whereas, in atrial fibrillation, an abnormal electrical signal into the atrium is transmitted very rapidly and arbitrarily, so that the atrium is unable to contract normally, and the very rapid electrical signal in the atrium is irregularly transmitted to the ventricle, whereby the heartbeat is fast and its intervals are very irregular.

The methods for treating the atrial fibrillation include drug treatment, radiofreqency catheter ablation, and implantable cardioverter defibrillators (ICD) implantation. Although 70% of patients with atrial fibrillation receive drug treatment, this treatment is not for complete recovery, but for reduction of the likelihood of paralysis caused by arrhythmia and for relief of the symptom. The radiofreqency catheter ablation is a treatment in which the tissue that causes arrhythmia is identified using a cardiac electrophysiology study and high radiofrequency is emitted to the tissue to destroy the causative tissue. In implantable cardioverter defibrillators Implantation, an implantable cardioverter defibrillator monitors whether atrial fibrillation occurs, by sensing an electrocardiogram, and transmits an electric current to perform defibrillation when atrial fibrillation occurs, thereby reducing the likelihood of recurrence. The defibrillator is implanted into the patient's body in the form of a pacemaker, and is composed of a defibrillator and a lead, which are similar composition to that of the pacemaker.

While conducting a clinical trial to treat mitral valve regurgitation by using a cerclage catheter of Korean Patent No. 10-1116867, Korean Patent No. 10-1563172, Korean Patent No. 10-1581021, and Korean Patent Application Publication No. 10-2017-0044065, the present inventor has observed a phenomenon, wherein the heart is returned to its normal rhythm as the size of the left atrium is reduced by tightening the base of the left ventricle. However, there remains a likelihood of recurrence. As part of this study, the present invention provides a device to treat valve regurgitation and atrial fibrillation recurring at the same time as the pacemaker lead is fixed.

The United States Patent No. 6,745,081 has filed a patent application for an intracardiac defibrillator to treat atrial fibrillation and ventricular fibrillation. However, since the intracardiac defibrillator is a device for treatment when atrial fibrillation and ventricular fibrillation occur, there is a problem in that atrial fibrillation and ventricular fibrillation may not be fundamentally eliminated. WO-A-2019/046205 describes an annuloplasty device can be placed in the coronary sinus to reshape the mitral valve and reduce mitral valve regurgitation. The disclosure also provides improved techniques for cardiac pacing.

### Disclosure

### Technical Problem

An objective of the present invention is to provide a device to treat atrial fibrillation, and to maximally increase a recovery effect to normal heartbeat by treating the recurrent atrial fibrillation.

Another objective of the present invention is to provide a device to treat mitral valve regurgitation and tricuspid valve regurgitation by using a single device, bradycardia by fixing a pacemaker lead, and atrial fibrillation by fixing a defibrillator lead.

The objectives of the present invention are not limited to the above-mentioned objectives, and other objectives that are not mentioned will be clearly understood by those skilled in the art from the following description.

### Technical Solution

In the present invention to achieve the above objectives, a cerclage atrial defibrillator according to claim 1 is provided.

The defibrillator lead may include a first defibrillation electrode positioned in the superior vena cava and a second defibrillation electrode positioned in the left atrium.

The defibrillator lead may include at a lower part thereof an electrocardiogram sensing electrode for sensing an electrical stimulus of the heart.

The defibrillator lead may be coupled to the coronary sinus tube.

A lower part of the coronary sinus tube may be coupled to a left atrium branch tube into which the second defibrillation electrode is to be inserted.

A cerclage atrial defibrillator according to another exemplary embodiment includes: a cerclage wire; a coronary sinus tube provided with a lumen formed therein into which the cerclage wire is inserted, the coronary sinus tube being inserted into the coronary sinus; a tricuspid valve tube provided with a lumen formed therein for inserting the cerclage wire, the tricuspid valve tube traversing the tricuspid valve and protecting the tricuspid valve and tissue of the interventricular septum; a pacemaker lead having an end thereof inserted into the bundle of His; a defibrillator lead insertion tube coupled to the coronary sinus tube and having an end thereof facing towards a wall of the superior vena cava; and a defibrillator lead positioned in the superior vena cava or the left atrium to treat atrial fibrillation.

The defibrillator lead may include a first defibrillation electrode positioned in the superior vena cava and a second defibrillation electrode positioned in the left atrium.

The defibrillator lead may include an electrocardiogram sensing electrode for sensing an electrical stimulus of the heart.

The first defibrillation electrode may be coupled to the defibrillator lead insertion tube.

The second defibrillation electrode may be coupled to the coronary sinus tube.

A lower part of the coronary sinus tube may be coupled to a left atrium branch tube into which the second defibrillation electrode is inserted.

In a cerclage atrial defibrillator,

the cerclage atrial defibrillator according to yet another exemplary embodiment includes: a cerclage wire; a coronary sinus tube provided with a lumen formed therein into which the cerclage wire is inserted, the coronary sinus tube being inserted into the coronary sinus; a tricuspid valve tube provided with a lumen formed therein, the tricuspid valve tube traversing the tricuspid valve and protecting the tricuspid valve and tissue of the interventricular septum; and a defibrillator lead positioned in the superior vena cava or the left atrium to treat atrial fibrillation.

The defibrillator lead may include a first defibrillation electrode positioned in the superior vena cava and a second defibrillation electrode positioned in the left atrium.

The defibrillator lead may include an electrocardiogram sensing electrode for sensing an electrical stimulus of the heart.

The defibrillator lead may be coupled to the coronary sinus tube.

The cerclage atrial defibrillator may further include: a defibrillator lead insertion tube coupled to the coronary sinus tube and having an end thereof facing towards a blood vessel wall of the superior vena cava.

The first defibrillation electrode may be coupled to the defibrillation lead insertion tube.

A lower part of the coronary sinus tube may be coupled to a left atrium branch tube into which the second defibrillation electrode is inserted.

The cerclage atrial defibrillator may further include: a pacemaker lead having an end thereof inserted into the bundle of His.

### Advantageous Effects

As described above, the cerclage atrial defibrillator according to the present invention may not only simply relieve atrial fibrillation that occurs, but may also reduce the occurrence of the atrial fibrillation by tightening the mitral valve by using a cerclage wire, whereby atrial fibrillation that often recurs may be recovered to normal heartbeat by treatment with the defibrillator.

In addition, the cerclage atrial defibrillator according to the present invention may be used for various purposes for mitral valve regurgitation treatment, tricuspid valve regurgitation treatment, fixation of a pacemaker lead for heart failure treatment, and fixation of a defibrillator lead for atrial fibrillation treatment.

### Description of Drawings

FIG. 1 is a perspective view of a cerclage atrial defibrillator according to a preferred exemplary embodiment of the present invention.
FIG. 2 is a perspective view of a defibrillator lead according to the preferred exemplary embodiment of the present invention.
FIG. 3 is a perspective view of a cerclage atrial defibrillator according to a second preferred exemplary embodiment of the present invention.
FIG. 4 is a perspective view of a cerclage atrial defibrillator according to a third preferred exemplary embodiment of the present invention.
FIG. 5 is a perspective view of a cerclage atrial defibrillator according to a fourth preferred exemplary embodiment of the present invention, FIG. 5(a) is a perspective view showing a case where a lower part of a defibrillator lead insertion tube is positioned in the right atrium, and FIG. 5(b) is a perspective view showing a case where the lower part of the defibrillator lead insertion tube is positioned in the superior vena cava.
FIG. 6 is a perspective view of a cerclage atrial defibrillator according to a fifth preferred exemplary embodiment of the present invention.
FIG. 7 is a perspective view of a cerclage atrial defibrillator according to a sixth preferred exemplary embodiment of the present invention.
FIG. 8 is a flowchart for a treatment method, which is excluded from the scope of the present invention, using the cerclage atrial defibrillator according to the preferred exemplary embodiments of the present invention.

### <Description of the Reference Numerals in the Drawings>

- 10:: cerclage wire
- 12:: arch part
- 20:: coronary sinus tube
- 30:: tricuspid valve tube
- 32:: blocking part
- 40:: left atrium branch tube
- 50:: pacemaker lead
- 60:: stopper
- 70:: defibrillator lead insertion tube
- 80:: defibrillator lead
- 82:: first defibrillation electrode
- 84:: second defibrillation electrode
- 86:: electrocardiogram sensing electrode

### Best Mode

Benefits and features of the present invention and methods of accomplishing the same may be understood more readily by reference to the following detailed description of exemplary embodiments and the accompanying drawings. However, the present invention is not limited to the exemplary embodiments disclosed below, but will only be defined by the appended claims.

Hereinafter, exemplary embodiments of the present invention will be described in detail with reference to the accompanying drawings. The same reference numerals refer to the same components regardless of the drawings, and "and / or" includes each and every combination of one or more of the mentioned items.

The terminology used herein is for the purpose of describing particular embodiments only and is not intended to be limiting of the invention. In this specification, the singular form also includes the plural form unless otherwise specified in the phrase. As used herein, "comprises" and / or "comprising" does not exclude the presence or addition of one or more other components in addition to the mentioned components.

Unless otherwise defined, all terms (including technical and scientific terms) used in the present description may be used in a sense that can be commonly understood by those skilled in the art. In addition, terms defined in the commonly used dictionary are not ideally or excessively interpreted unless specifically defined.

Hereinafter, the preferred exemplary embodiments of the present invention will be described in detail with reference to the accompanying drawings.

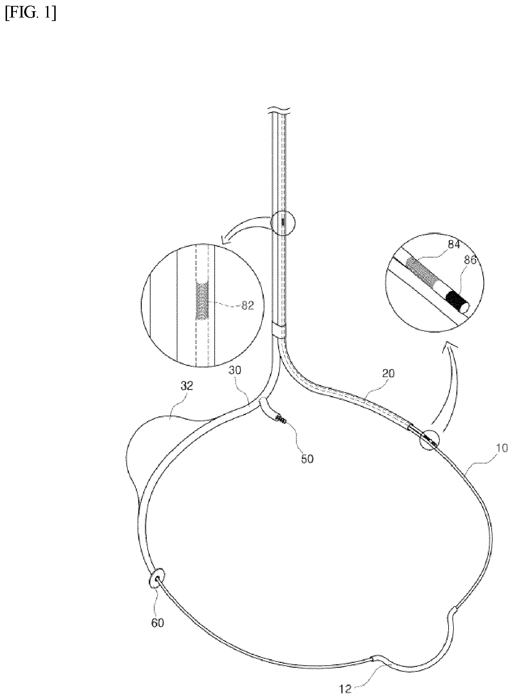

FIG. 1 is a perspective view of a cerclage atrial defibrillator according to a preferred exemplary embodiment of the present invention.

Referring to FIG. 1, the cerclage atrial defibrillator according to the preferred exemplary embodiment of the present invention fundamentally includes a cerclage wire 10, a coronary sinus tube 20, a tricuspid valve tube 30, a pacemaker lead 50, and a defibrillation lead 80.

The cerclage wire 10 is a single thread that is inserted into the coronary vein, is passed through the interventricular septum, and is passed through the tricuspid valve, whereby the name is given in the sense that the single thread passes through a part of the coronary sinus, the interventricular septum, and a part of the tricuspid valve in sequence, and then circling back like drawing a circle, and is discharged from a patient's body. When pushing or pulling the cerclage wire 10 from the top, the cerclage wire serves to maintain proper tension force according to the size and shape of a patient's heart, and to tighten the mitral annulus. Subsequently, mitral valve regurgitation may be treated. According to the results of clinical trials of the present inventor, when the mitral valve is tightened, the occurrence of atrial fibrillation in a patient with the atrial fibrillation is reduced and may be treated.

The cerclage wire 10 further includes an arch part 12, and the arch part 12 is formed on one side of the cerclage wire 10. When the cerclage wire 10 is inserted into the coronary sinus, the arch part is to prevent the external pressure applied to the coronary artery positioned below the coronary sinus and to protect the coronary artery.

The coronary sinus tube 20 is provided with a lumen formed therein into which the cerclage wire 10 may be inserted. The upper part of the coronary sinus tube 20 is connected to the tricuspid valve tube 30 and is formed in a straight line, and the lower part thereof is in a curved shape to be inserted into the coronary sinus. The coronary sinus tube 20 is inserted into the coronary sinus to protect the coronary sinus.

The tricuspid valve tube 30 is provided with a lumen formed therein into which the cerclage wire 10 may be inserted. The tricuspid valve tube 30 is passed through an orifice generated due to an incomplete closing of the tricuspid valve and reaches the interventricular septum, and is provided with a stopper 60 installed at the lower part thereof to prevent the end of the tricuspid valve tube 30 from penetrating into the interventricular septum. By making the tricuspid valve tube 30 float around the tricuspid valve without contacting with the tricuspid valve, the stopper 60 is able to prevent damage to the tricuspid valve due to the tricuspid valve tube 30.

The tricuspid valve tube 30 includes a blocking part 32. The blocking part 32 is a part that blocks the orifice generated due to an incomplete closing of the tricuspid valve, and may be a blocking membrane, a blocking balloon, or the like. By blocking the orifice of the tricuspid valve with the blocking part, tricuspid valve regurgitation may be treated.

The pacemaker lead 50 is inserted into the tricuspid valve tube 30 or coupled to the tricuspid valve tube 30, and is provided with the lower part thereof facing the interventricular septum. The pacemaker lead 50 may perform sensing and pacing of an electrical signal of the heart. The pacemaker lead 50 includes a direction adjustment device (not shown) at the upper part thereof, moves the lower part of the pacemaker lead 50 by adjusting the direction adjustment device (not shown), may detect an electrocardiogram of the interventricular septum to identify the position of the bundle of His, and fixes the end of the pacemaker lead 50 to the position of the detected bundle of His. The pacemaker lead 50 may not be included depending on the purpose of using the device of the present invention.

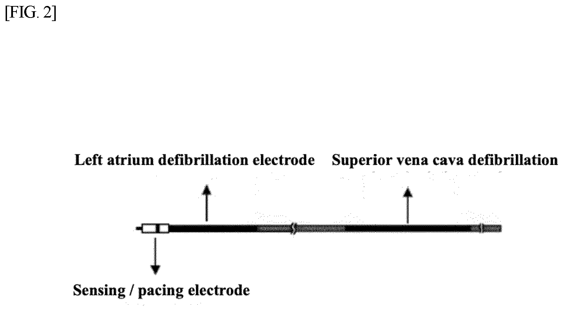

FIG. 2 is a perspective view of a defibrillator lead according to the preferred exemplary embodiment of the present invention.

Referring to FIG. 2, in a single lead, the defibrillator lead 80 includes a first defibrillation electrode 82, a second defibrillation electrode 84, and an electrocardiogram sensing electrode 86. The outer part of the defibrillator lead 80 is made of an insulator excluding the first defibrillation electrode 82, the second defibrillation electrode 84, and the electrocardiogram sensing electrode 86, and thus current does not flow therethrough. The first defibrillation electrode 82 and the second defibrillation electrode 84 are preferably formed in a coil shape, but are not limited thereto. The electrocardiogram sensing electrode 86 is coupled to the lower end of the defibrillator lead 80 so as to perform sensing that detects the electrical signal of the heart and transmits the signal to the defibrillator, and to perform a function of pacing that transmits the current received from the defibrillator to the heart as needed.

Referring to FIG. 1, the first defibrillation electrode 82 is positioned in the superior vena cava, and the second defibrillation electrode 84 is positioned in the coronary sinus in the left atrium to perform defibrillation when atrial fibrillation occurs. More particularly, the defibrillator lead 80 is inserted into and coupled to the coronary sinus tube 20, the first defibrillation electrode 82 is positioned at the upper part of the coronary sinus tube 20 to contact the superior vena cava, and the second defibrillation electrode 84 is positioned at the lower part of the coronary sinus tube 20 to contact the left atrium. Because of not being an insulator, the coronary sinus tube 20 is able to transmit electricity when in contact with the superior vena cava and the left atrium, even when the first defibrillation electrode 82 and the second defibrillation electrode 84 are positioned inside the coronary sinus tube 20. It is apparent that the defibrillator lead 80 may be coupled to the outer circumferential surface of the coronary sinus tube 20.

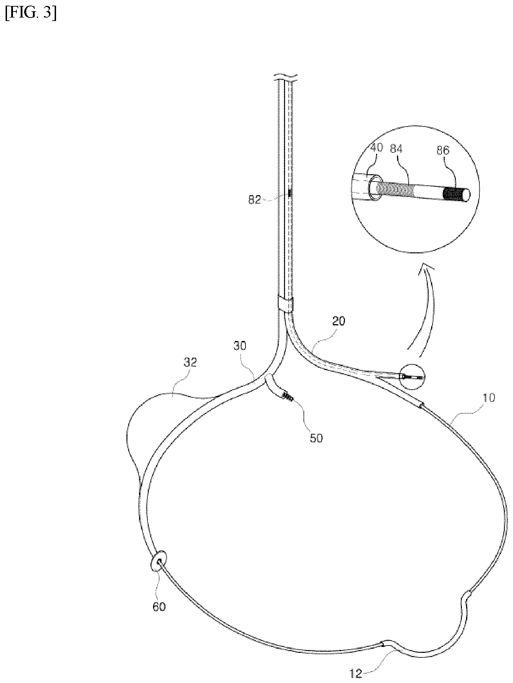

FIG. 3 is a perspective view of a cerclage atrial defibrillator according to a second preferred exemplary embodiment of the present invention.

Referring to FIG. 3, the cerclage atrial defibrillator according to the second preferred exemplary embodiment of the present invention includes a cerclage wire 10, a coronary sinus tube 20, a tricuspid valve tube 30, a left atrium branch tube 40, a pacemaker lead 50, and a defibrillator lead 80.

One end of the left atrium branch tube 40 is coupled to one side of the coronary sinus tube 20, and the other end thereof is directed toward the coronary sinus and in communication with the coronary sinus tube 20. The defibrillator lead 80 is as described above in FIG. 2. The lower part of the defibrillator lead 80 is inserted into the left atrium branch tube 40. The second defibrillation electrode 84 is positioned inside the left atrium branch tube 40, or is positioned outside thereof by passing through the left atrium branch tube 40 and is contacted with the left atrium, thereby performing defibrillation to the left atrium when atrial fibrillation occurs.

Configurations other than the left atrium branch tube 40 are as described above with reference to FIGS. 1 and 2.

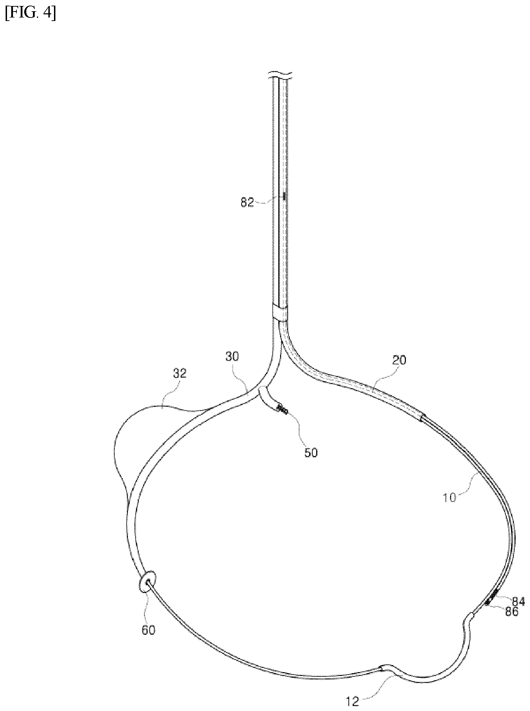

FIG. 4 is a perspective view of a cerclage atrial defibrillator according to a third preferred exemplary embodiment of the present invention.

Referring to FIG. 4, the cerclage atrial defibrillator according to the third preferred exemplary embodiment of the present invention includes a cerclage wire 10, a coronary sinus tube 20, a tricuspid valve tube 30, a pacemaker lead 50, and a defibrillator lead 80.

The defibrillator lead 80 is the same as described above in FIG. 2. The second defibrillation electrode 84 is inserted into the coronary sinus tube 20, and may be passed through the coronary sinus tube 20 to directly contact the coronary sinus. In this case, the defibrillator lead 80 may further include a fixation device (not shown) for fixing the defibrillator lead 80 at an end thereof, which may be in the form of a screw, but is not limited thereto. The fixation device (not shown) is for fixing the second defibrillation electrode 84, which is in the coronary sinus, to reach a desired position.

In the third preferred exemplary embodiment of the present invention, the configuration other than those described above is the same as described above in FIGS. 1 and 2.

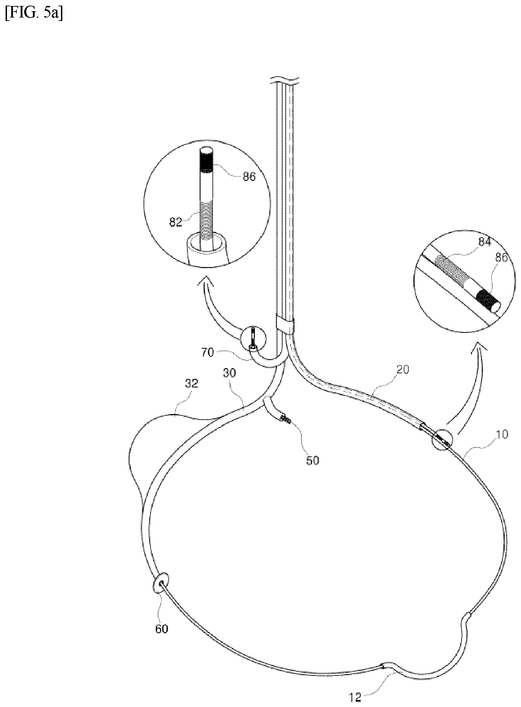

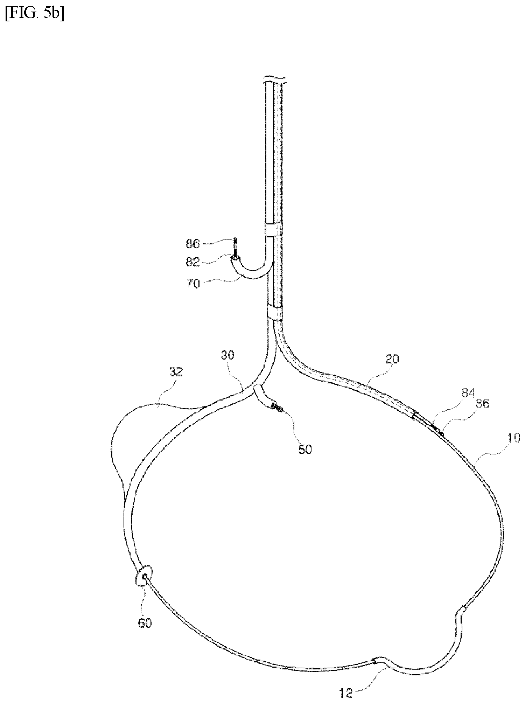

FIG. 5 is a perspective view of a cerclage atrial defibrillator according to a fourth preferred exemplary embodiment of the present invention, FIG. 5(a) is a perspective view showing a case where a lower part of a defibrillator lead insertion tube is positioned in the right atrium, and FIG. 5(b) is a perspective view showing a case where the lower part of the defibrillator lead insertion tube is positioned in the superior vena cava.

Referring to FIG. 5, the cerclage atrial defibrillator according to the fourth preferred exemplary embodiment of the present invention includes a cerclage wire 10, a coronary sinus tube 20, a tricuspid valve tube 30, a pacemaker lead 50, a defibrillator lead insertion tube 70, and a defibrillator lead 80.

Referring to FIGS. 5(a) and 5(b), the defibrillator lead insertion tube 70 is provided with a lumen formed therein into which the defibrillator lead 80 may be inserted, is provided with the upper part thereof connected to the coronary sinus tube 20, and is provided with the lower part thereof branched so that the end thereof is curved to face the wall of the superior vena cava or the right atrium.

The defibrillator lead 80 of the cerclage atrial defibrillator according to the fourth preferred exemplary embodiment of the present invention is composed of two leads including a first defibrillation electrode 82 and a second defibrillation electrode 84, in which each lead includes an electrocardiogram sensing electrode 86 at each of the lower part thereof.

The lead including the first defibrillation electrode 82 is inserted into and fixed to the defibrillator lead insertion tube 70. The first defibrillation electrode 82 is positioned at the lower part of the defibrillator lead insertion tube 70, or is passed through the defibrillator lead insertion tube 70, and is fixed to contact a position of the wall of the superior vena cava or the right atrium that a surgeon desires. The end of the lead including the first defibrillation electrode 82 may further include a fixation device (not shown) for fixing the lead, and the fixation device may be in the form of a screw, but is not limited thereto.

The lead including the second defibrillation electrode 84 is inserted into and fixed to the coronary sinus tube 20, and the second defibrillation electrode 84 is positioned at the lower part of the coronary sinus tube 20.

The configuration other than those described about the cerclage atrial defibrillator according to the fourth preferred exemplary embodiment of the present invention is the same as described above in FIG. 1.

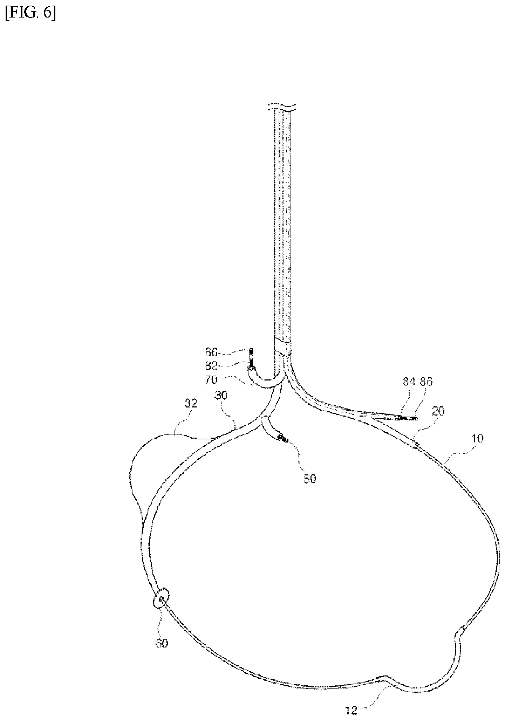

FIG. 6 is a perspective view of a cerclage atrial defibrillator according to a fifth preferred exemplary embodiment of the present invention.

Referring to FIG. 6, a cerclage atrial defibrillator according to the fifth preferred exemplary embodiment of the present invention includes a cerclage wire 10, a coronary sinus tube 20, a tricuspid valve tube 30, a left atrium branch tube 40, a pacemaker lead 50, a defibrillator lead insertion tube 70, and a defibrillator lead 80.

One end of the left atrium branch tube 40 is coupled to one side of the coronary sinus tube 20, and the other end thereof is directed toward the coronary sinus and in communication with the coronary sinus tube 20. The defibrillator lead insertion tube 70 is as described above in FIG. 5.

Two defibrillator leads 80 are configured as described above in FIG. 5, and the lead including the first defibrillation electrode 82 is inserted into and fixed to the defibrillator lead insertion tube 70. The first defibrillation electrode 82 is positioned at the lower part of the defibrillator lead insertion tube 70, or is passed through the defibrillator lead insertion tube 70, and is fixed to contact a position of the wall of the superior vena cava or the right atrium that a surgeon desires. The lead including the second defibrillation electrode 84 is inserted into the left atrium branch tube 40, and the second defibrillation electrode 84 is positioned in the left atrium branch tube 40, or is passed through the left atrium branch tube 40, and is fixed to contact a position of the coronary sinus that a surgeon desires. A fixation device (not shown) may be coupled to the end of the lead including the left atrium branch tube 40 to fix the defibrillator lead 80, and the fixation device may be in the form of a screw, but is not limited thereto.

The configuration other than those described about the cerclage atrial defibrillator according to the fifth preferred exemplary embodiment of the present invention is the same as described above in FIG. 1.

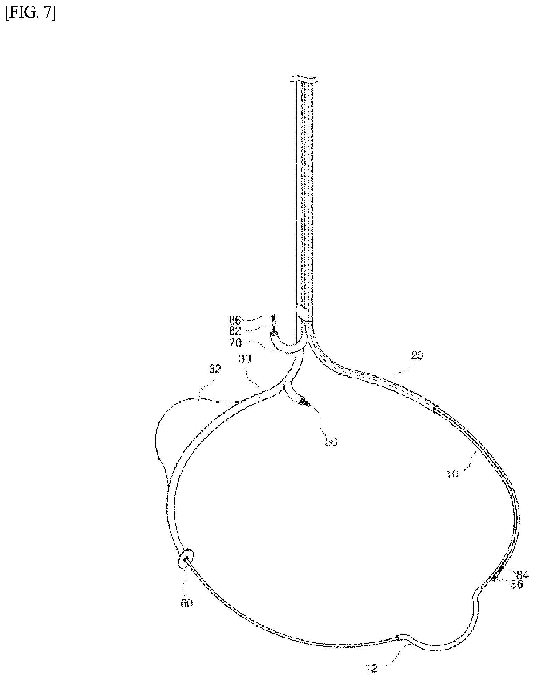

FIG. 7 is a perspective view of a cerclage atrial defibrillator according to a sixth preferred exemplary embodiment of the present invention.

Referring to FIG. 7, the cerclage atrial defibrillator according to the sixth preferred exemplary embodiment of the present invention includes a cerclage wire 10, a coronary sinus tube 20, a tricuspid valve tube 30, a pacemaker lead 50, a defibrillator lead insertion tube 70, and a defibrillator lead 80.

The defibrillator lead insertion tube 70 is as described above in FIGS. 5 and 6. Two defibrillator lead 80 are composed of a lead including the first defibrillation electrode 82 and a lead including the second defibrillation electrode 84, as described above with reference to FIG. 5, and each of the electrocardiogram sensing electrodes 86 is coupled to each of the lower part of the two leads. The first defibrillation electrode 82 is positioned at the lower part of the defibrillator lead insertion tube 70, or is passed through the defibrillator lead insertion tube 70, and is fixed to contact a position of the wall of the superior vena cava or the right atrium that a surgeon desires. The end of the lead including the first defibrillation electrode 82 may further include a fixation device (not shown) for fixing the lead, and the fixation device may be in the form of a screw, but is not limited thereto. The lead including the second defibrillation electrode 84 is inserted into the coronary sinus tube 20, and the second defibrillation electrode 84 is passed through the coronary sinus tube 20, and is fixed to contact a position of the coronary sinus that a surgeon desires. The end of the lead including the second defibrillation electrode 84 may further include a fixation device (not shown) for fixing the lead, and the fixation device may be in the form of a screw, but is not limited thereto.

The configuration other than those described about the cerclage atrial defibrillator according to the sixth preferred exemplary embodiment of the present invention is the same as described above in FIG. 1.

The cerclage atrial defibrillator according to the above-described exemplary embodiments may not only simply relieve atrial fibrillation that occurs, but may also reduce the occurrence of atrial fibrillation by tightening the mitral valve by using a cerclage wire, whereby atrial fibrillation that often recurs may be restored to normal heartbeat by treatment with the defibrillator.

In addition, with a single device, mitral valve regurgitation treatment, tricuspid valve regurgitation treatment, fixation of the pacemaker lead for bradycardia heart failure treatment, and insertion of the defibrillator lead for atrial fibrillation treatment may be performed.

In addition, the device may also be used for at least one of the four treatments described above.

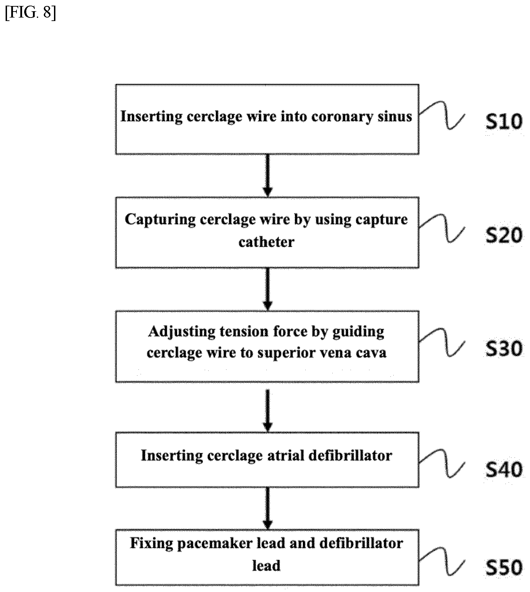

FIG. 8 is a flowchart for a treatment method using the cerclage atrial defibrillator according to the preferred exemplary embodiments of the present invention.

First, a step S10 is for inserting the cerclage wire into the coronary sinus is for moving the cerclage wire 10 through the superior vena cava, the coronary sinus, the septal vein, and the right ventricle in sequence.

In order to facilitate the movement of the cerclage wire 10, a balloon catheter (not shown) may be included. The balloon catheter (not shown) blocks blood flow in the coronary sinus to make the coronary sinus to be swollen, thereby allowing the septal vein, which is generally difficult to identify, to be easily seen.

The cerclage wire 10 is passed through the interventricular septum via the septal vein, and moves to the right ventricle. In order to pass through the interventricular septum, the cerclage wire 10 is provided with a sharp end thereof, but when it is difficult to penetrate the interventricular septum with the cerclage wire 10 by itself, a perforating catheter (not shown) is required. The perforating catheter (not shown) serves to support when the cerclage wire 10 perforates the interventricular septum, thereby allowing the cerclage wire 10 to pass through the interventricular septum more easily.

Next, a step S20 is for capturing the cerclage wire by using a capture catheter. The capture catheter (not shown) is provided with a mesh net formed thereon, and the mesh net may be folded or unfolded by force applied to the capture catheter (not shown) in vitro. The capture catheter (not shown) moves through the inferior vena cava, the tricuspid valve, and the right ventricle, and may further include a safe-zone-passing catheter (not shown), in order for the capture catheter (not shown) to safely move in the patient's body. When moving to the right ventricle, the mesh net is folded and then unfolded in the right ventricle, and the cerclage wire 10 is inserted into the mesh net. The mesh net is folded again, and after capturing the cerclage wire 10, the mesh net is extracted out of the patient's body through the inferior vena cava. At this time, one end of the cerclage wire 10 is positioned in the superior vena cava, and the other end thereof is positioned in the inferior vena cava.

Next, a step S30 is for adjusting tension force by guiding the cerclage wire to the superior vena cava. The cerclage wire 10 guided to the inferior vena cava is guided back to the superior vena cava by a snare, forceps, or the like, which is inserted from the superior vena cava, and both ends of the cerclage wire are positioned in vitro through the superior vena cava. Therefore, the cerclage wire 10 is passed through the superior vena cava, the coronary sinus, the septal vein, the interventricular septum, and the right ventricle in sequence, and is positioned in the superior vena cava again, thereby coming back like drawing a circular shape, so that both ends of the cerclage wire are positioned in vitro. While pushing or pulling the cerclage wire 10, appropriate tension force is maintained according to the size and shape of the patient's heart, and the mitral annulus is tightened to hold, and thus mitral valve regurgitation is treated by enabling the mitral valve to be completely closed.

Next, a step S40 is for inserting a cerclage atrial defibrillator. After respectively inserting both ends of the cerclage wire 10 outside the patient's body into the coronary sinus tube 20 and the tricuspid valve tube 30, the coronary sinus tube 20 and the tricuspid valve tube 30 are inserted into the patient's body along the cerclage wire 10. At this time, the pacemaker lead 50 coupled to the tricuspid valve tube 30 is inserted in the right atrium direction together with the tricuspid valve tube 30, and the defibrillator lead 80 coupled to the coronary sinus tube 20 is inserted into the superior vena cava and the coronary sinus.

The stopper 60 is coupled to the lower end of the tricuspid valve tube 30, and may be coupled to the lower part of the tricuspid valve tube 30. This allows the tricuspid valve tube 30 to float around the tricuspid valve without being in close contact therewith, thereby preventing damage to the tricuspid valve. In addition, the tricuspid valve tube 30 includes the blocking part 32, which penetrates into the orifice generated due to an incomplete closing phenomenon of the tricuspid valve to completely close the tricuspid valve. The blocking part 32 may be in the form of a blocking membrane, a blocking balloon, or the like.

Finally, a step S50 is for fixing the pacemaker lead and the defibrillator lead is performed. The pacemaker lead 50 may be adjusted to bend or unfold the lower part thereof by installing a direction adjustment device 54 at the upper part thereof. The lower part of the pacemaker lead 50 is moved by the direction adjustment device 54, detects an electrocardiogram of the interventricular septum to identify the position of the bundle of His, and fixes the end of the pacemaker lead 50 to the detected position of the bundle of His.

When the first defibrillation electrode 82 and the second defibrillation electrode 84 of the defibrillator lead 80 are positioned within the coronary sinus tube 20, a separate fixation device (not shown) is unnecessary, thereby coupling to positions in the coronary sinus tube 20 and transmitting electrical stimulation by contacting the superior vena cava and the left atrium. However, in the case where the first defibrillation electrode 82 and the second defibrillation electrode 84 are positioned outside the coronary sinus tube 20 and are in direct contact with the superior vena cava and the left ventricle, the fixation device (not shown) may be further included in order to fix the electrodes to positions at which a surgeon desires.

The present invention is set out in the appended claims. The embodiments, examples or aspects according to the present description which do not fall within the scope of said claims are provided for illustrative purposes only and do not form part of the present invention.

## Claims

1. A cerclage atrial defibrillator, comprising:
a cerclage wire (10);
a coronary sinus tube (20) provided with a lumen formed therein which is adapted for insertion of the cerclage wire (10) thereinto, the coronary sinus tube (20) is adapted for being inserted into the coronary sinus; and
a tricuspid valve tube (30) provided with a lumen formed therein, wherein the tricuspid valve tube is adapted for traversing the tricuspid valve and adapted for protecting the tricuspid valve and tissue of the interventricular septum;
**characterized in that** the cerclage atrial defibrillator further comprises:
a defibrillator lead (80) adapted to be positioned in the superior vena cava or the left atrium to treat atrial fibrillation.

2. The cerclage atrial defibrillator according to claim 1, further comprising:
a pacemaker lead (50) adapted for having an end thereof inserted into the bundle of His.

3. the cerclage atrial defibrillator according to claim 2, further comprising:
a defibrillator lead insertion tube (70) coupled to the coronary sinus tube (20) and having an end thereof facing towards a wall of the superior vena cava, wherein the tricuspid valve tube (30) provided with a lumen formed therein is adapted for inserting therein the cerclage wire (10).

4. The cerclage atrial defibrillator of anyone of claims 1 to 3, wherein the defibrillator lead (80) comprises at a lower part thereof an electrocardiogram sensing electrode (86) adapted for sensing an electrical stimulus of the heart.

5. The cerclage atrial defibrillator of anyone of the preceding claims, wherein a lower part of the coronary sinus tube (20) is coupled to a left atrium branch tube into which the second defibrillation electrode (84) is adapted to be inserted.

6. The cerclage atrial defibrillator of anyone of the preceding claims, wherein the defibrillator lead (80) comprises a first defibrillation electrode (82) adapted to be positioned in the superior vena cava and a second defibrillation electrode (84) adapted to be positioned in the left atrium.

7. The cerclage atrial defibrillator of claim 6, wherein the first defibrillation electrode (82) is coupled to the defibrillator lead insertion tube (70).

8. The cerclage atrial defibrillator of anyone of claims 6 or 7, wherein the second defibrillation electrode (84) is coupled to the coronary sinus tube (20).

9. The cerclage atrial defibrillator of anyone of claims 6 to 8, wherein a lower part of the coronary sinus tube (20) is coupled to a left atrium branch tube into which the second defibrillation electrode (84) is inserted.

10. The cerclage atrial defibrillator of anyone of the preceding claims, wherein the defibrillator lead (80) comprises an electrocardiogram sensing electrode (86) for sensing an electrical stimulus of the heart.

11. The cerclage atrial defibrillator of anyone of the preceding claims wherein the defibrillator lead (80) is coupled to the coronary sinus tube (20).

12. The cerclage atrial defibrillator of anyone of claims 6 to 11 wherein the first defibrillation electrode (82) is coupled to the defibrillation lead insertion tube (70).

## Patentansprüche

1. Atrialer Cerclage-Defibrillator, umfassend:
einen Cerclage-Draht (10);
ein Koronarsinusrohr (20), das mit einem darin gebildeten Lumen versehen ist und das zum Einführen des Cerclage-Drahts (10) geeignet ist, wobei das Koronarsinusrohr (20) zum Einführen in den Koronarsinus geeignet ist; und
ein Trikuspidalklappenrohr (30), das mit einem darin gebildeten Lumen versehen ist, wobei das Trikuspidalklappenrohr geeignet ist, die Trikuspidalklappe zu durchqueren, und geeignet ist, um die Trikuspidalklappe und das Gewebe des Kammerseptums zu schützen;
**dadurch gekennzeichnet, dass** der atriale Cerclage-Defibrillator weiterhin umfasst:
einen Defibrillator-Anschlussdraht (80), der geeignet ist, ihn in der Vena cava superior oder der linken Herzkammer zu positionieren, um Kammerflimmern zu behandeln.

2. Atrialer Cerclage-Defibrillator gemäß Anspruch 1, weiterhin umfassend:
einen Schrittmacher-Anschlussdraht (50), der geeignet ist, eines seiner Enden in das His-Bündel einzustecken.

3. Atrialer Cerclage-Defibrillator gemäß Anspruch 2, weiterhin umfassend:
ein Defibrillator-Anschlussdraht-Einsteckrohr (70), das an das Koronarsinusrohr (20) gekoppelt ist und dessen eines Ende auf eine Wand der Vena cava superior gerichtet ist, wobei das Trikuspidalklappenrohr (30), das mit einem darin gebildeten Lumen versehen ist, geeignet ist, den Cerclage-Draht (10) einzuführen.

4. Atrialer Cerclage-Defibrillator gemäß einem der Ansprüche 1 bis 3, wobei der Defibrillator-Anschlussdraht (80) an einem unteren Teil davon eine Elektrokardiogramm-Sensorelektrode (86) umfasst, die geeignet ist, einen elektrischen Reiz des Herzens zu erfassen.

5. Atrialer Cerclage-Defibrillator gemäß einem der vorstehenden Ansprüche, wobei ein unterer Teil des Koronarsinusrohrs (20) an ein linkes Kammerzweigrohr gekoppelt ist, in das die zweite Defibrillationselektrode (84) angepasst ist um eingeführt zu werden.

6. Atrialer Cerclage-Defibrillator gemäß einem der vorstehenden Ansprüche, wobei der Defibrillator-Anschlussdraht (80) eine erste Defibrillationselektrode (82), die geeignet ist, sie in der Vena cava superior zu positionieren, und eine zweite Defibrillationselektrode (84), die geeignet ist, sie in der linken Herzkammer zu positionieren, umfasst.

7. Atrialer Cerclage-Defibrillator gemäß Anspruch 6, wobei die erste Defibrillationselektrode (82) an das Defibrillator-Anschlussdraht-Einsteckrohr (70) gekoppelt ist.

8. Atrialer Cerclage-Defibrillator gemäß einem der Ansprüche 6 oder 7, wobei die zweite Defibrillationselektrode (84) an das Koronarsinusrohr (20) gekoppelt ist.

9. Atrialer Cerclage-Defibrillator gemäß einem der Ansprüche 6 bis 8, wobei ein unterer Teil des Koronarsinusrohrs (20) an ein linkes Kammerzweigrohr gekoppelt ist, in das die zweite Defibrillationselektrode (84) eingesteckt wird.

10. Atrialer Cerclage-Defibrillator gemäß einem der vorstehenden Ansprüche, wobei der Defibrillator-Anschlussdraht (80) eine Elektrokardiogramm-Sensorelektrode (86) umfasst, um einen elektrischen Reiz des Herzens zu erfassen.

11. Atrialer Cerclage-Defibrillator gemäß einem der vorstehenden Ansprüche, wobei der Defibrillator-Anschlussdraht (80) an das Koronarsinusrohr (20) gekoppelt ist.

12. Atrialer Cerclage-Defibrillator gemäß einem der Ansprüche 6 bis 11, wobei die erste Defibrillationselektrode (82) an das Defibrillator-Anschlussdraht-Einsteckrohr (70) gekoppelt ist.

## Revendications

1. Défibrillateur atrial à cerclage, comprenant :
un fil de cerclage (10) ;
un tube de sinus coronaire (20) pourvu d'une lumière formée à l'intérieur qui est adaptée pour une insertion du fil de cerclage (10) dans celui-ci, le tube de sinus coronaire (20) est adapté pour être inséré dans le sinus coronaire ; et
un tube de valve tricuspide (30) pourvu d'une lumière formée à l'intérieur, dans lequel le tube de valve tricuspide est adapté pour traverser la valve tricuspide et adapté pour protéger la valve tricuspide et du tissu du septum interventriculaire ;
**caractérisé en ce que** le défibrillateur atrial à cerclage comprend en outre :
un fil conducteur de défibrillateur (80) adapté pour être positionné dans la veine cave supérieure ou l'atrium gauche pour traiter une fibrillation atriale.

2. Défibrillateur atrial à cerclage selon la revendication 1, comprenant en outre :
un fil conducteur de stimulateur cardiaque (50) adapté pour avoir une extrémité de celui-ci insérée dans le faisceau de His.

3. Défibrillateur atrial à cerclage selon la revendication 2, comprenant en outre :
un tube d'insertion de fil conducteur de défibrillateur (70) couplé au tube de sinus coronaire (20) et ayant une extrémité de celui-ci tournée vers une paroi de la veine cave supérieure, dans lequel le tube de valve tricuspide (30) pourvu d'une lumière formée à l'intérieur est adapté pour être inséré dans le fil de cerclage (10).

4. Défibrillateur atrial à cerclage selon l'une quelconque des revendications 1 à 3, dans lequel le fil conducteur de défibrillateur (80) comprend, au niveau d'une partie inférieure de celui-ci, une électrode de captage d'électrocardiogramme (86) adaptée pour capter un stimulus électrique du cœur.

5. Défibrillateur atrial à cerclage selon l'une quelconque des revendications précédentes, dans lequel une partie inférieure du tube de sinus coronaire (20) est couplée à un tube de dérivation d'atrium gauche dans lequel la seconde électrode de défibrillation (84) est adaptée pour être insérée.

6. Défibrillateur atrial à cerclage selon l'une quelconque des revendications précédentes, dans lequel le fil conducteur de défibrillateur (80) comprend une première électrode de défibrillation (82) adaptée pour être positionnée dans la veine cave supérieure et une seconde électrode de défibrillation (84) adaptée pour être positionnée dans l'atrium gauche.

7. Défibrillateur atrial à cerclage selon la revendication 6, dans lequel la première électrode de défibrillation (82) est couplée au tube d'insertion de fil conducteur de défibrillateur (70).

8. Défibrillateur atrial à cerclage selon l'une quelconque des revendications 6 ou 7, dans lequel la seconde électrode de défibrillation (84) est couplée au tube de sinus coronaire (20).

9. Défibrillateur atrial à cerclage selon l'une quelconque des revendications 6 à 8, dans lequel une partie inférieure du tube de sinus coronaire (20) est couplée à un tube de dérivation d'atrium gauche dans lequel la seconde électrode de défibrillation (84) est insérée.

10. Défibrillateur atrial à cerclage selon l'une quelconque des revendications précédentes, dans lequel le fil conducteur de défibrillateur (80) comprend une électrode de captage d'électrocardiogramme (86) pour capter un stimulus électrique du cœur.

11. Défibrillateur atrial à cerclage selon l'une quelconque des revendications précédentes, dans lequel le fil conducteur de défibrillateur (80) est couplé au tube de sinus coronaire (20).

12. Défibrillateur atrial à cerclage selon l'une quelconque des revendications 6 à 11, dans lequel la première électrode de défibrillation (82) est couplée au tube d'insertion de fil conducteur de défibrillation (70).
